# EUROPEAN PATENT APPLICATION

(11) **EP 4 477 110 A1**
(43) Date of publication of application: **18.12.2024**
(21) Application number: 23183899.6
(22) Date of filing: 06.07.2023
(51) Int. Cl.: A46B 15/00, A61B 5/00, A61B 5/107

(54) **ESTIMATING HEAD TILT ANGLE OF TOOTHBRUSH USER**

(30) Priority: 12.06.2023 US 202363472566 P
(71) Applicant: Koninklijke Philips N.V., 5656 AG Eindhoven (NL)
(72) Inventor: VIJAYKUMAR, Adithya, Eindhoven (NL); VLUTTERS, Ruud, Eindhoven (NL); RIETMAN, Ronald, Eindhoven (NL)
(74) Representative: Philips Intellectual Property & Standards

(57) **Abstract**

The subject-matter of the present disclosure relates to a computer-implemented method of estimating a head tilt angle of a user of a toothbrush during brushing, the computer-implemented method comprising: sensing, using a sensor of the toothbrush, movement parameters associated with the toothbrush over a brushing time period; inputting, to a machine learning model, the sensed movement parameters to estimate a head tilt angle of the user at each time point of the brushing time period; and generating the head tilt angle of the user as the head tilt angle estimated by the first machine learning model.

## Description

### FIELD OF THE INVENTION

The subject-matter of the present disclosure relates to a computer-implemented method of estimating a head tilt angle of a user of a toothbrush during brushing, computer-implemented method of training a machine learning model to estimate a segment of a dental arch of a user being brushed by a user of a toothbrush, a computer-implemented method of training a machine learning model to estimate a head tilt angle of a user of the toothbrush, and a transitory, or non-transitory, computer-readable medium.

### BACKGROUND OF THE INVENTION

Modern toothbrushes include a body, a head having bristles, an inertial measurement unit (IMU) in the body, and a controller for monitoring and controlling various features of the toothbrush. For example, a toothbrush may provide an alert when the head requires changing after a certain number of uses, or an alert that the head may need replacing if the toothbrush has been dropped.

Such functions are governed by algorithms stored in a storage of the controller and executed by a processor of the controller. Some of those algorithms require inputs associated with various parameters of the toothbrush. Some inputs can be sensed directly from the IMU, such as angular velocity. However, some parameters are not readily available. For example, head tilt angle of the user is not possible to measure using the IMU.

It is an aim of the subject-matter of the present disclosure to improve on the prior art.

### SUMMARY OF THE INVENTION

According to a first aspect of the present invention, there is provided a computer-implemented method of estimating a head tilt angle of a user of a toothbrush during brushing, the computer-implemented method comprising: sensing, using a sensor of the toothbrush, movement parameters associated with the toothbrush over a brushing time period; inputting, to a machine learning model, the sensed movement parameters to estimate a head tilt angle of the user at each time point of the brushing time period; and generating the head tilt angle of the user as the head tilt angle estimated by the machine learning model. Advantageously, the machine learning model enables the head tilt angle to be determined based on readily available movement parameters.

In an embodiment, the machine learning model comprises a long short-term memory recurrent neural network, LSTM.

In an embodiment, the machine learning model comprises an encoder between an input layer and the LSTM and comprises a decoder between the LSTM and an output layer, wherein the encoder comprises a convolutional neural network, wherein the classifier comprises a convolutional neural network.

In an embodiment, the movement parameters include acceleration and angular velocity and the sensor is an inertial measurement unit.

According to another aspect of the present invention, there is provided a computer-implemented method of training a machine learning model to estimate a head tilt angle of a user of the toothbrush, the computer-implemented method comprising: receiving a training data set and a head tilt ground truth, wherein, for each time point of a brushing time period, the training data set includes movement parameter values and the head tilt ground truth includes a ground truth head tilt angles'; estimating, using the machine learning model, a head tilt angle for each time point of the brushing time period using the respective movement parameter values as inputs; calculating a loss between the estimated brushing angles and the ground truth head tilt angles'; and adjusting a parameterisation of the machine learning model to minimise the loss.

In an embodiment, the computer-implemented method further comprises: generating the head tilt ground truth by: capturing a video of a user brushing their teeth with a toothbrush over a sample period, a marker on the user's head and a marker on the toothbrush, sensing, using a sensor of the toothbrush, movement parameter values at each time point of the sample period, calculating, at each time point of the sample period, a head tilt angle using an orientation of the marker, and generating, for each time point, the head tilt angle associated with the movement parameter values; and sending the head tilt angle ground truth to the machine learning model.

In an embodiment, the calculating a loss between the estimated head tilt angles and the ground truth head tilt angles' comprises, for each time point of the brushing time period, determining a ground truth unit vector, vgt, and an estimated head tilt angle unit vector, veba, by respectively calculating cosine and sine components of the head tilt angles in the ground truth and the estimated head tilt angles', and calculating the loss, L, using *L* = 1 - *v_{gt}. v_{eba}*, where . denotes an inner dot product between vgt and veba.

In an embodiment, the computer-implemented method further comprises: training a machine learning model (60) to estimate a segment of a dental arch of a user being brushed by a user of a toothbrush, the computer-implemented method comprising: receiving a training data set and a segment ground truth, wherein, for each time point of a brushing time period, the training data set includes movement parameter values and the segment ground truth head tilt angles indicates a segment of a dental arch being brushed; estimating, using the machine learning model, a brushing segment for each time point of the brushing time period using the respective movement parameter values as inputs; calculating a loss between the estimated brushing segments and the segments indicated by the labels; and adjusting a parameterisation of the machine learning model to minimize the loss; re-initialising parameters associated with a final layer of the machine learning model; and fine-tuning the machine learning model by the: estimating, using the machine learning model, a head tilt angle for each time point of the brushing time period using the respective movement parameter values as inputs; calculating a loss between the estimated head tilt angles and the ground truth head tilt angles'; and adjusting a parameterisation of the machine learning model to minimise the loss.

In an embodiment, the computer-implemented method further comprises: generating the segment ground truth by: capturing a video of a user brushing their teeth using a toothbrush over a sample period, sensing, using a sensor of the toothbrush, movement parameter values at each time point of the sample period, displaying the video to a user of a user interface, receiving a plurality of user inputs indicating a brushing segment currently being brushed by the toothbrush in the video, and labelling each time point of the sample period using the segments from the plurality of user inputs; and sending the ground truth to the machine learning model.

In an embodiment, the inputting, to a machine learning model, the sensed movement parameters to estimate a segment of the a dental arch being contacted by the bristles at each time point of the brushing time period comprises inputting, to the machine learning model, the sensed movement parameters to estimate a segment of the a dental arch being contact by the bristles from amongst a list a segments of a dental arch including upper front facial, and upper front lingual, upper right facial, upper right occlusal, upper right lingual, upper left facial, upper left occlusal, upper left lingual, lower front facial, and lower front lingual, lower right facial, lower right occlusal, lower right lingual, lower left facial, lower left occlusal, lower left lingual.

According to an aspect of the present invention, there is provided a transitory, or non-transitory, computer-readable medium having instructions stored thereon that, when executed by a computer, cause the computer to perform the computer-implemented method of any preceding aspect or embodiment.

These and other aspects of the present invention will be apparent from and elucidated with reference to the embodiment(s) described hereinafter.

### BRIEF DESCRIPTION OF DRAWINGS

The embodiments of the present invention may be best understood with reference to the accompanying figures, in which:
Fig. 1 shows a schematic block diagram of a toothbrush according to one or more embodiments;
Fig. 2 shows an illustration of upper and lower dental arches of a user of the toothbrush of Fig. 1;
Fig. 3 shows a block diagram of a machine learning model trained to estimate a head tilt angle of a user of the toothbrush according to one or more embodiments;
Fig. 4 shows a block diagram of a machine learning model trained to estimate a segment of a dental arch being brushed by the toothbrush form Fig. 1 according to one or more embodiments;
Fig. 5 shows a schematic of a set-up of a training laboratory for collecting training data for a ground truth for training the machine learning model from Fig. 3;
Fig. 6 shows a schematic of a toothbrush for use in the training laboratory from Fig. 5;
Fig. 7 shows a schematic of a head marker for use in the training laboratory from Fig. 5;
Fig. 8 shows a flow chart summarising steps associated with a computer-implemented method of estimating a head tilt angle of a user of a toothbrush during brushing using the machine learning model of Fig. 3 according to one or more embodiments; and
Fig. 9 shows a flow chart summarising steps associated with a computer-implemented method of training the machine learning model from Fig. 3 according to one or more embodiments.

### DESCRIPTION OF EMBODIMENTS

At least some of the example embodiments described herein may be constructed, partially or wholly, using dedicated special-purpose hardware. Terms such as 'component', 'module' or 'unit' used herein may include, but are not limited to, a hardware device, such as circuitry in the form of discrete or integrated components, a Field Programmable Gate Array (FPGA) or Application Specific Integrated Circuit (ASIC), which performs certain tasks or provides the associated functionality. In some embodiments, the described elements may be configured to reside on a tangible, persistent, addressable storage medium and may be configured to execute on one or more processors. These functional elements may in some embodiments include, by way of example, components, such as software components, object-oriented software components, class components and task components, processes, functions, attributes, procedures, subroutines, segments of program code, drivers, firmware, microcode, circuitry, data, databases, data structures, tables, arrays, and variables. Although the example embodiments have been described with reference to the components, modules and units discussed herein, such functional elements may be combined into fewer elements or separated into additional elements. Various combinations of optional features have been described herein, and it will be appreciated that described features may be combined in any suitable combination. In particular, the features of any one example embodiment may be combined with features of any other embodiment, as appropriate, except where such combinations are mutually exclusive. Throughout this specification, the term "comprising" or "comprises" means including the component(s) specified but not to the exclusion of the presence of others.

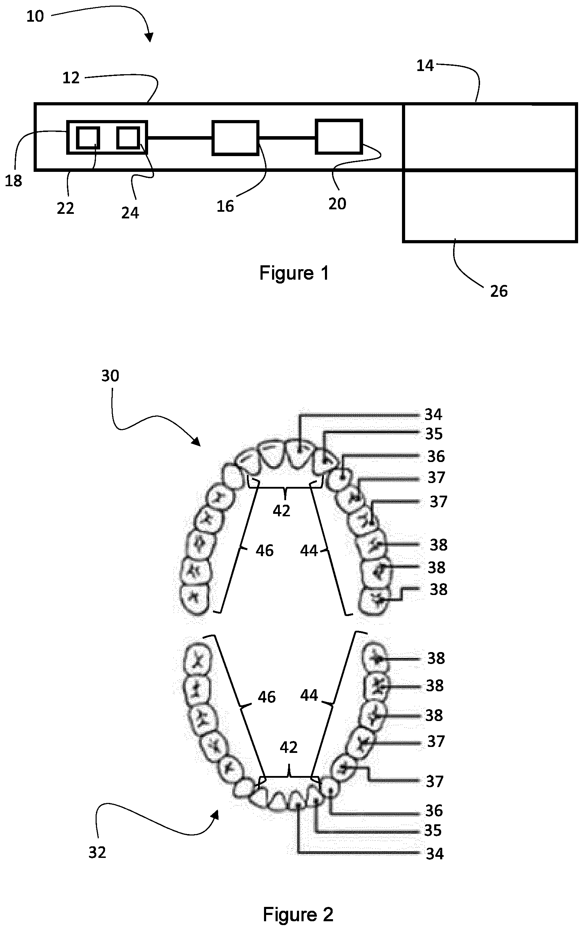

With reference to Fig. 1, a toothbrush 10 includes an elongate body forming a handle 12, and a head 14 at an end of the handle 12. The toothbrush also includes an energy storage module 16, a controller 18, and a sensor 20 within the handle.

The energy storage module 16 may be a battery. The battery may be a secondary, or rechargeable, battery. The controller may include a processor 22 and storage 24. The computer-implemented methods described below may be embodied as electronic data defining instructions stored in the storage as non-transitory computer-readable media that when executed by the processor cause the processor to perform the respective computer-implemented method. The instructions may be loaded onto the storage and thus be embodied as transitory computer readable media.

The sensor 20 may be an inertial measurement unit (IMU) sensor. The IMU sensor may be configured to sense rotational velocity and acceleration. The rotational velocity and acceleration may be sensed in three axes, which may be cartesian axes including x, y, and z axes. The energy storage module 16 may be connected to the controller 18 and the sensor 20 to power them both.

The head 14 may include bristles 26 for brushing a user's teeth.

With reference to Fig. 2, a user's teeth may be categorised into an upper dental arch 30 and a lower dental arch 32. Each of the upper and lower dental arches should include the following tooth types: central incisor 34, lateral incisor 35, canine 36, premolar 37, and molar 38. Each tooth type other than the central and lateral incisors 34, 35, includes a facial side, a lingual side, and an occlusal side. The central and lateral incisors include a facial side and a lingual side, yet no occlusal side.

The dental arches may be split into different segments. The tooth types may be grouped within the segments. There may be sixteen segments. There may be eight segments in the upper dental arch 30 and eight segments in the lower dental arch 32. The segments may include two segments at a front 42 of each dental arch, three segments at a right 44 side of each dental arch, and three segments at a left 46 side of each dental arch. The central and lateral incisors 34, 35, are within the front 42 of each dental arch. The interface between the incisors (e.g. the lateral incisors) and the canines 36 may be the interface between the front and left/right 42, 44 segments.

In this way, the segments include upper front facial, and upper front lingual, upper right facial, upper right occlusal, upper right lingual, upper left facial, upper left occlusal, upper left lingual, lower front facial, and lower front lingual, lower right facial, lower right occlusal, lower right lingual, lower left facial, lower left occlusal, and lower left lingual.

In other embodiments, there may be a different number of segments, and the segments may include a different number of teeth. For example, there may be a segment definition where each segment includes a single tooth.

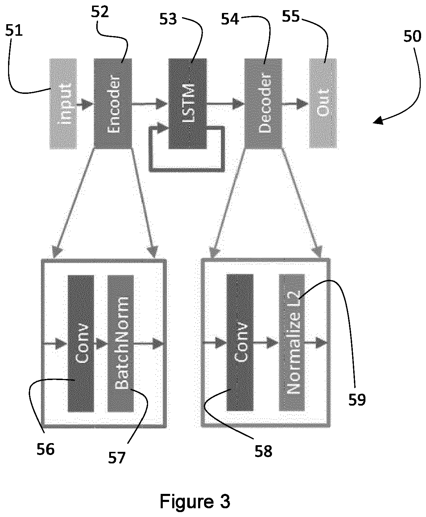

With reference to Fig. 3, a machine learning model is provided for estimating the head tilt angle of a user of a toothbrush during brushing at each time point of a brushing time period using sensed movement parameters as inputs. This machine learning model may be called a first machine learning model 50.

The first machine learning model may include a recurrent neural network, which may be a long short-term memory recurrent neural network, LSTM. The first machine learning model 50 comprises an input 51, an encoder 52, the LSTM 53, a decoder 54, and an output 55. The encoder 52 includes a convolutional neural network 56 (or convolution layer) and a BatchNorm layer 57. The decoder 54 includes a convolutional neural network 58 (or convolution layer) and a normalise L2 layer 59.

In the encoder 52, the input movement parameters get filtered (linearly) by the convolution layer and scaled by the batch normalization layer. This filtered signal goes into the LSTM layer that updates its internal (also called hidden) state. Next, the hidden state of the LSTM is used as input to the decoder, that maps the hidden state into two channels (using a convolution layer with 2 output channels, and a kernel size of 1). As last step, these 2 channels are normalized to represent a vector of length 1.

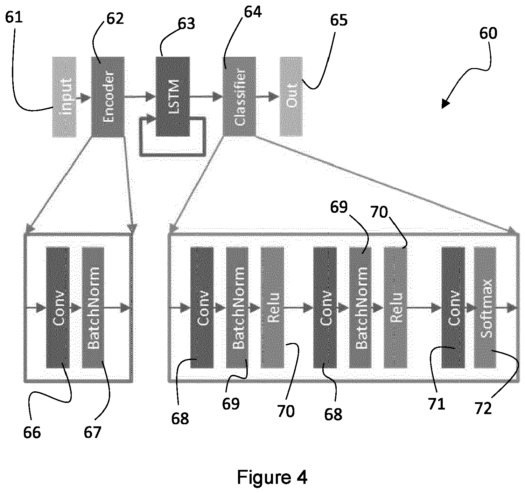

With reference to Fig. 4, a second machine learning model 60 is provided for estimating a segment of a dental arch being contacted by the bristles at each time point of the brushing time period using the movement data as inputs.

The second machine learning model 60 includes an input 61, an encoder 62, an LSTM 63, a classifier 64, and an output 65. The encoder 62 includes a convolutional neural network 66 (or convolution layer) and a BatchNorm layer 67. The classifier 64 includes a first group of three layers including a convolutional neural network 68 (convolution layer), a BatchNorm layer 69, and an activation function 70, and a second group including the same three layers in the same order as the first group. The activation function 70 may be a ReLu activation function. The classifier 64 also includes a convolutional neural network 71 (or convolution layer) and a softmax layer 72. The softmax layer includes a plurality of nodes representing a probability distribution, where a total of the probabilities of the probabilities of all of the plurality of nodes equals 1.

To train the first and second machine learning models, first the second machine learning model is trained.

The segment ground truth may be generated by capturing a video of a user brushing their teeth using a toothbrush over a sample period, sensing, using a sensor of the toothbrush, movement parameter values at each time point of the sample period, displaying the video to the user of the toothbrush using a user interface, receiving a plurality of user inputs indicating a segment of a dental arch currently being brushed by the toothbrush in the video, and the user of the toothbrush then manually labelling each time point of the sample period using the segments from the plurality of user inputs. The IMU data, or movement parameters of each time point, may be taken as a training data set and the ground truth include the labels indicating the segment of the dental arch being brushed.

The method of training the machine learning model includes receiving the training data set and the segment ground truth, estimating, using the machine learning model, a brushing segment for each time point of the brushing time period using the respective movement parameter values as inputs, calculating a loss between the estimated brushing segments and the segments indicated by the labels, and adjusting a parameterisation of the machine learning model to minimise the loss. The adjusting the parameters may be carried out using an optimisation algorithm. The parameterisation may refer to weights throughout the machine learning model.

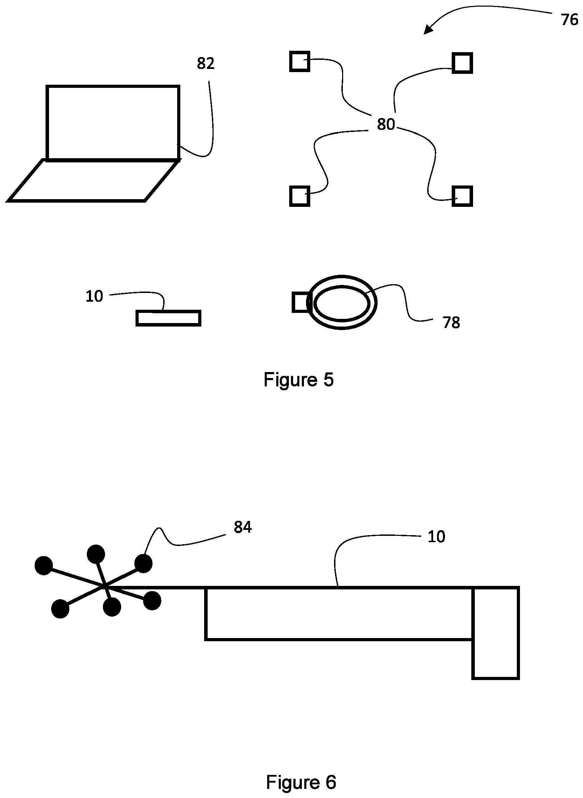

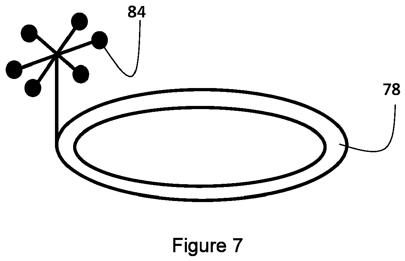

With reference to Figs. 5 to 7, another aspect relates to a computer-implemented method of training the first machine learning model to estimate a head tilt angle of the user of the toothbrush. The method first requires collection of training data and generating a corresponding ground truth. The training data may be collected in a laboratory 76 where the ground truth is constructed.

The laboratory includes a toothbrush 10, a headset 78, cameras 80, and a computing system 82. The toothbrush 10 includes a plurality of markers 84 fixed thereto. The markers may be detected by the cameras 80 so that an orientation of the toothbrush can be detected. The headset may also include a plurality of markers 86 which may be detected by the cameras 80 to deduce a head tilt angle of the user.

The method may comprise, generating the head tilt ground truth by: capturing a video of a user brushing their teeth with a toothbrush over a sample period, the user having a marker on their head; sensing, using a sensor of the toothbrush, movement parameter values at each time point of the sample period; calculating, at each time point of the sample period, a head tilt angle using an orientation of the marker, and generating, for each time point, a ground truth head tilt angle associated with the movement parameter values; and sending the head tilt angle ground truth to the machine learning model.

The method may comprise receiving a training data set and a head tilt ground truth, wherein, for each time point of a brushing time period, the training data set includes movement parameter values and the head tilt ground truth includes a ground truth head tilt angle; estimating, using the machine learning model, a head tilt angle for each time point of the brushing time period using the respective movement parameter values as inputs; calculating a loss between the estimated brushing angles and the ground truth head tilt angles; and adjusting a parameterisation of the machine learning model to minimise the loss. The adjustment may be performed using an optimisation algorithm, and the parameterisation may refer to weights of the machine learning model.

The calculating a loss between the estimated brushing angles and the ground truth brushing angles, for each time point of the brushing time period, determining a ground truth unit vector, v_{gt}, and an estimated brushing angle unit vector, v_{eba}, by respectively calculating cosine and sine components of the brushing angles in the ground truth and the estimated brushing angles, and calculating the loss, L, using *L* = 1 - *v_{gt}. v_{eba}*, where . denotes an inner dot product between v_{gt} and v_{eba}.

During the training procedure, the IMU data will be used as input, and the brushing angle as ideal / ground-truth that we want to obtain. Hence, when an AI model predicts the brushing angle, one can use this ideal brushing angle with the predicted brushing angle and minimize the difference. As it would be desirable to predict angles, it is desirable that the metric being minimized (also called the loss function) converges. When naively using a simple mean absolute difference in angles, one might suffer from the circular definition of these angles, where an angle of -179 is only 2 degrees away from an angle of +179 degree. To solve this, it is possible to predict the cosine and sine components of the brushing angle (effectively a vector with length = 1) and convert the ground truth also to the cosine and sine of the ground truth angle.

It should be noted that whilst it is possible to train the first machine learning model using randomised initialisation of weights, it may be better in certain circumstances to start with the trained second machine learning model and use transfer learning. This because the ground truth and training data for training the second machine learning model does not have to be carried out in the laboratory. Instead of the cameras used in the laboratory, a smart phone of a user can be used outside the context of the laboratory.

To train the first machine learning model the method may comprise providing the second machine learning model trained using the computer-implemented method described above; re-initialising parameters associated with a final layer of the machine learning model; and fine-tuning the machine learning model by the: estimating, using the machine learning model, a brushing angle for each time point of the brushing time period using the respective movement parameter values as inputs; calculating a loss between the estimated brushing angles and the ground truth brushing angles; and adjusting a parameterisation of the machine learning model to minimise the loss.

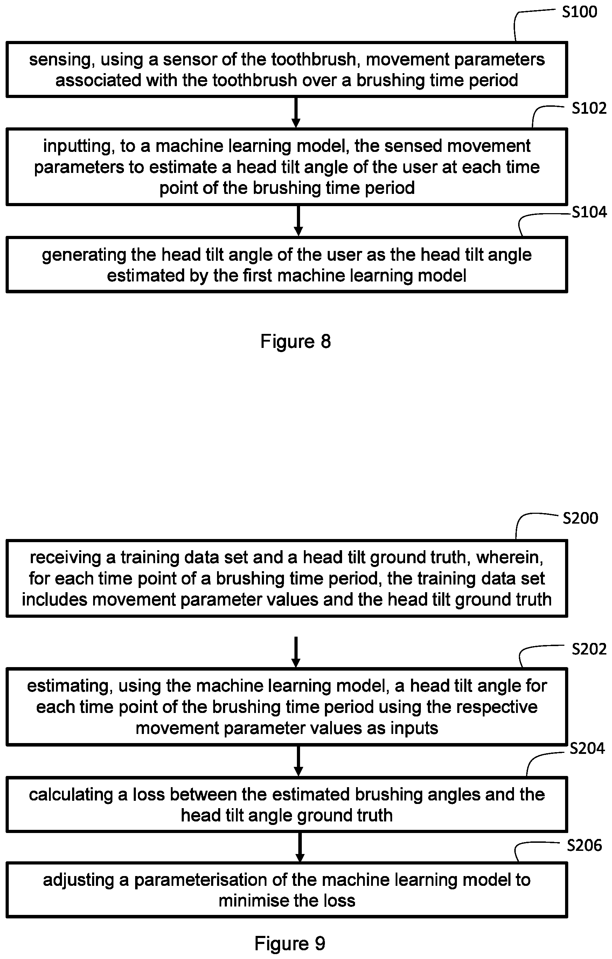

With reference to Fig. 8, the computer-implemented method of estimating a head tilt angle of a user of a toothbrush during brushing may be summarised as including: sensing S 100, using a sensor of the toothbrush, movement parameters associated with the toothbrush over a brushing time period; inputting S 102, to a machine learning model, the sensed movement parameters to estimate a head tilt angle of the user at each time point of the brushing time period; and generating S 104 the head tilt angle of the user as the head tilt angle estimated by the first machine learning model.

With reference to Fig. 9, the computer-implemented method of training a machine learning model to estimate a head tilt angle of a user of the toothbrush may be summarised as including: receiving S200 a training data set and a head tilt ground truth, wherein, for each time point of a brushing time period, the training data set includes movement parameter values and the ground truth head tilt angles; estimating S202, using the machine learning model, a head tilt angle for each time point of the brushing time period using the respective movement parameter values as inputs; calculating S204 a loss between the estimated brushing angles and the ground truth head tilt angles; and adjusting S206 a parameterisation of the machine learning model to minimise the loss.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measured cannot be used to advantage. Any reference signs in the claims should not be construed as limiting the scope.

Various features disclosed herein may be summarised in the following clauses.

Clause 1. A computer-implemented method of estimating a head tilt angle of a user of a toothbrush during brushing, the computer-implemented method comprising: sensing, using a sensor of the toothbrush, movement parameters associated with the toothbrush over a brushing time period; inputting, to a machine learning model, the sensed movement parameters to estimate a head tilt angle of the user at each time point of the brushing time period; and generating the head tilt angle of the user as the head tilt angle estimated by the machine learning model.

Clause 2. The computer-implemented method of Clause 1, wherein the machine learning model comprises a long short-term memory recurrent neural network, LSTM.

Clause 3. The computer-implemented method of Clause 2, wherein the machine learning model comprises an encoder between an input layer and the LSTM and comprises a decoder between the LSTM and an output layer, wherein the encoder comprises a convolutional neural network, wherein the classifier comprises a convolutional neural network.

Clause 4. The computer-implemented method of Clause 1, wherein the movement parameters include acceleration and angular velocity and the sensor is an inertial measurement unit.

Clause 5. A computer-implemented method of training a machine learning model to estimate a head tilt angle of a user of the toothbrush, the computer-implemented method comprising: receiving a training data set and a head tilt ground truth, wherein, for each time point of a brushing time period, the training data set includes movement parameter values and the head tilt ground truth includes a ground truth head tilt angles'; estimating, using the machine learning model, a head tilt angle for each time point of the brushing time period using the respective movement parameter values as inputs; calculating a loss between the estimated brushing angles and the ground truth head tilt angles'; and adjusting a parameterisation of the machine learning model to minimise the loss.

Clause 6. The computer-implemented method of Clause 5, further comprising: generating the head tilt ground truth by: capturing a video of a user brushing their teeth with a toothbrush over a sample period, marker on each of the user's head and the toothbrush; sensing, using a sensor of the toothbrush, movement parameter values at each time point of the sample period, calculating, at each time point of the sample period, a head tilt angle using an orientation of the marker, and generating, for each time point, a ground truth head tilt angle associated with the movement parameter values; and sending the head tilt angle ground truth to the machine learning model.

Clause 7. The computer-implemented method of Clause 5, wherein the calculating a loss between the estimated head tilt angles and the ground truth head tilt angles' comprises, for each time point of the brushing time period, determining a ground truth unit vector, v_{gt}, and an estimated head tilt angle unit vector, v_{eba}, by respectively calculating cosine and sine components of the head tilt angles in the ground truth and the estimated head tilt angles', and calculating the loss, L, using *L* = 1 - *v_{gt}. v_{eba}*, where . denotes an inner dot product between v_{gt} and v_{eba}.

Clause 8. The computer-implemented method of Clause 5, further comprising: training a machine learning model (60) to estimate a segment of a dental arch of a user being brushed by a user of a toothbrush, the computer-implemented method comprising: receiving a training data set and a segment ground truth, wherein, for each time point of a brushing time period, the training data set includes movement parameter values and the segment ground truth head tilt angles indicates a segment of a dental arch being brushed; estimating, using the machine learning model, a brushing segment for each time point of the brushing time period using the respective movement parameter values as inputs; calculating a loss between the estimated brushing segments and the segments indicated by the labels; and adjusting a parameterisation of the machine learning model to minimize the loss; re-initialising parameters associated with a final layer of the machine learning model; and fine-tuning the machine learning model by the: estimating, using the machine learning model, a head tilt angle for each time point of the brushing time period using the respective movement parameter values as inputs; calculating a loss between the estimated head tilt angles and the ground truth head tilt angles'; and adjusting a parameterisation of the machine learning model to minimise the loss.

Clause 9. The computer-implemented method of Clause 8, further comprising: generating the segment ground truth by: capturing a video of a user brushing their teeth using a toothbrush over a sample period, sensing, using a sensor of the toothbrush, movement parameter values at each time point of the sample period, displaying the video to a user of a user interface, receiving a plurality of user inputs indicating a brushing segment currently being brushed by the toothbrush in the video, and labelling each time point of the sample period using the segments from the plurality of user inputs; and sending the ground truth to the machine learning model.

Clause 10. The computer-implemented method of Clause 9, wherein the inputting, to a machine learning model, the sensed movement parameters to estimate a segment of the a dental arch being contacted by the bristles at each time point of the brushing time period comprises inputting, to the machine learning model, the sensed movement parameters to estimate a segment of the a dental arch being contact by the bristles from amongst a list a segments of a dental arch including upper front facial, and upper front lingual, upper right facial, upper right occlusal, upper right lingual, upper left facial, upper left occlusal, upper left lingual, lower front facial, and lower front lingual, lower right facial, lower right occlusal, lower right lingual, lower left facial, lower left occlusal, lower left lingual.

## Claims

1. A computer-implemented method of estimating a head tilt angle of a user of a toothbrush (10) during brushing, the computer-implemented method comprising:
sensing (S 100), using a sensor of the toothbrush, movement parameters associated with the toothbrush over a brushing time period;
inputting (S102), to a machine learning model (50), the sensed movement parameters to estimate a head tilt angle of the user at each time point of the brushing time period;
and
generating (S104) the head tilt angle of the user as the head tilt angle estimated by the machine learning model.

2. The computer-implemented method of Claim 1, wherein the machine learning model comprises a long short-term memory recurrent neural network, LSTM (53).

3. The computer-implemented method of Claim 2, wherein the machine learning model comprises an encoder (52) between an input layer (51) and the LSTM and comprises a decoder (54) between the LSTM and an output layer (55), wherein the encoder comprises a convolutional neural network (56), wherein the classifier comprises a convolutional neural network (58).

4. The computer-implemented method of any preceding claim, wherein the movement parameters include acceleration and angular velocity and the sensor is an inertial measurement unit.

5. A computer-implemented method of training a machine learning model (50) to estimate a head tilt angle of a user of the toothbrush (10), the computer-implemented method comprising:
receiving (S200) a training data set and a head tilt ground truth, wherein, for each time point of a brushing time period, the training data set includes movement parameter values and the head tilt ground truth includes a ground truth head tilt angles';
estimating (S202), using the machine learning model, a head tilt angle for each time point of the brushing time period using the respective movement parameter values as inputs;
calculating (S204) a loss between the estimated brushing angles and the ground truth head tilt angles'; and
adjusting (S206) a parameterisation of the machine learning model to minimise the loss.

6. The computer-implemented method of Claim 5, further comprising:
generating the head tilt ground truth by:
capturing a video of a user brushing their teeth with a toothbrush over a sample period, a marker on each of the user's head and the toothbrush,
sensing, using a sensor (20) of the toothbrush, movement parameter values at each time point of the sample period,
calculating, at each time point of the sample period, a head tilt angle using an orientation of the marker, and
generating, for each time point, a head tilt angle ground truth associated with the movement parameter values; and
sending the head tilt angle ground truth to the machine learning model.

7. The computer-implemented method of any of Claims 5 to 6, wherein the calculating a loss between the estimated head tilt angles and the ground truth head tilt angles' comprises, for each time point of the brushing time period, determining a ground truth unit vector, v_{gt}, and an estimated head tilt angle unit vector, v_{eba}, by respectively calculating cosine and sine components of the head tilt angles in the ground truth and the estimated head tilt angles', and calculating the loss, L, using *L* = 1 - *v_{gt}. v_{eba}*, where . denotes an inner dot product between v_{gt} and v_{eba}.

8. The computer-implemented method of any of Claims 5 to 7, further comprising:
training a machine learning model (60) to estimate a segment of a dental arch of a user being brushed by a user of a toothbrush, the computer-implemented method comprising:
receiving a training data set and a segment ground truth, wherein, for each time point of a brushing time period, the training data set includes movement parameter values and the segment ground truth includes a label indicating a segment of a dental arch being brushed;
estimating, using the machine learning model, a brushing segment for each time point of the brushing time period using the respective movement parameter values as inputs;
calculating a loss between the estimated brushing segments and the segments indicated by the labels; and
adjusting a parameterisation of the machine learning model to minimize the loss;
re-initialising parameters associated with a final layer of the machine learning model; and
fine-tuning the machine learning model by the:
estimating, using the machine learning model, a head tilt angle for each time point of the brushing time period using the respective movement parameter values as inputs;
calculating a loss between the estimated head tilt angles and the ground truth head tilt angles'; and
adjusting a parameterisation of the machine learning model to minimise the loss.

9. The computer-implemented method of Claim 8, further comprising:
generating the segment ground truth by:
capturing a video of a user brushing their teeth using a toothbrush over a sample period, sensing, using a sensor of the toothbrush, movement parameter values at each time point of the sample period,
displaying the video to a user of a user interface,
receiving a plurality of user inputs indicating a brushing segment currently being brushed by the toothbrush in the video, and
labelling each time point of the sample period using the segments from the plurality of user inputs;
and sending the ground truth to the machine learning model.

10. The computer-implemented method of Claim 9, wherein the inputting, to a machine learning model, the sensed movement parameters to estimate a segment of the a dental arch being contacted by the bristles at each time point of the brushing time period comprises inputting, to the machine learning model, the sensed movement parameters to estimate a segment of the a dental arch being contact by the bristles from amongst a list a segments of a dental arch including upper front facial, and upper front lingual, upper right facial, upper right occlusal, upper right lingual, upper left facial, upper left occlusal, upper left lingual, lower front facial, and lower front lingual, lower right facial, lower right occlusal, lower right lingual, lower left facial, lower left occlusal, lower left lingual.
